# EUROPEAN PATENT APPLICATION

(11) **EP 4 509 089 A1**
(43) Date of publication of application: **19.02.2025**
(21) Application number: 23191545.5
(22) Date of filing: 15.08.2023
(51) Int. Cl.: A61B 90/20, A61B 46/00, A61B 46/10

(54) **SURGICAL IMAGING DEVICE, SURGICAL IMAGING SYSTEM AND METHOD FOR VENTILATION**

(71) Applicant: Leica Instruments (Singapore) Pte Ltd, Singapore 608924 (SG)
(72) Inventor: FRICK, Roman, 9435 Heerbrugg (CH); GAECHTER, Leander, 9435 Heerbrugg (CH)
(74) Representative: Recknagel, Stefan

(57) **Abstract**

The present disclosure relates to a surgical imaging device, a surgical imaging system and a method for ventilation of a surgical imaging device. The surgical imaging device comprises an image acquisition apparatus carrier, an arm, a stand, and a ventilation system. The ventilation system comprises an air intake, a fan, a flow path, and an exhaust part. The ventilation system is configured to generate an air flow inside the surgical imaging device. The fan is positioned within the flow path configured to create a directed air flow along the flow path from the air intake to the exhaust part. An intake port of the air intake is positioned at a first end of the arm. The exhaust part is positioned in the stand. The flow path extends from the intake port though the arm and the stand to the exhaust part.

## Description

### Technical field

The present disclosure relates to a surgical imaging device, a surgical imaging system and a method for ventilation of a surgical imaging device.

### Background

In an operating room, surgical imaging devices are typically covered at least in part by a drape. The drape shields sensitive components of the surgical imaging device, such as an optics carrier or a camera, from mechanical or material impacts during surgery, for example scraping with tools or contamination with liquid spills. The drape may also be easily cleaned and thereby contributes to maintaining a sterile environment in the operating room.

As the drape mantles parts of the microscope which are near the operating site, however, further efforts are required to ensure close-fitting of the drape and avoid disturbance of medical personnel during surgery.

### Summary

It is therefore a desire for an improved concept for a surgical imaging device allowing for improved fitting of a drape with minimal disturbance of a user.

This desire is addressed by the subject-matter of the independent claims.

Embodiments of the present disclosure provide a surgical imaging device. The surgical imaging device comprises an image acquisition apparatus carrier, an arm, a stand, and a ventilation system. The ventilation system comprises an air intake, a fan, a flow path, and an exhaust part. The ventilation system is configured to generate an air flow inside the surgical imaging device. The fan is positioned within the flow path configured to create a directed air flow along the flow path from the air intake to the exhaust part. An intake port of the air intake is positioned at a first end of the arm. The exhaust part is positioned in the stand. The flow path extends from the intake port though the arm and the stand to the exhaust part.

In an operating room, a drape may be mounted onto the surgical imaging device covering at least parts of the image acquisition apparatus carrier, the arm and/or the stand. By that the air flow into the air intake may reduce an air volume inside of the drape thereby reducing an overall volume of the drape and increasing a room to maneuver of a user, such as a surgeon or surgery assistant. The air flow is guided from the air intake at the end of the arm, which is a position close to an operating site, to the exhaust part in the stand, which is a position with a great distance to the operating site. The exhaust air is thereby directed away from the user, who may perform a medical procedure at the operating site. Hence, the user remains undisturbed by the exhaust air. Components of the ventilation system may be configured to be attached to or positioned inside the surgical imaging device for taking up no or less additional space. Thereby a free moving space of the user is maintained and the operating room is kept clear of further equipment.

Embodiments of the present disclosure further provide a surgical imaging system. The surgical imaging system is comprising the surgical imaging device and a detachable drape. The drape is thereby configured to partly cover the surgical imaging device.

Embodiments of the present disclosure further provide a corresponding method for ventilation of a surgical imaging device. The method comprises the following steps: Powering up of the surgical imaging device. Initiating the fan at a high-power state thereby creating an air flow suitable for reducing an air volume inside a drape, which is at least partly covering the surgical imaging device. Reaching a reduced air volume inside the drape. Switching the fan to a second power state suitable for maintaining the reduced air volume inside the drape. The second power state of the fan and/or a power-on state of a second fan of the ventilation system is thereby suited to maintain the air flow along the flow path.

### Short Description of the Figures

Some examples of apparatuses and/or methods will be described in the following by way of example only, and with reference to the accompanying figures, in which
- Fig. 1: shows a schematic perspective view of a surgical imaging device according to an embodiment;
- Fig. 2: shows a schematic side view of the surgical imaging device;
- Fig. 3: shows a schematic sectional view of a connector piece of an arm of the surgical imaging device according to an embodiment;
- Fig. 4: shows a schematic side view of the connector piece;
- Fig. 5: shows a schematic sectional view of a sub-arm of the arm according to an embodiment;
- Fig. 6: shows a schematic sectional view of a stand of the surgical imaging device according to an embodiment;
- Fig. 7: shows a flow chart of a method for ventilation of a surgical imaging device according to an embodiment.

### Detailed Description

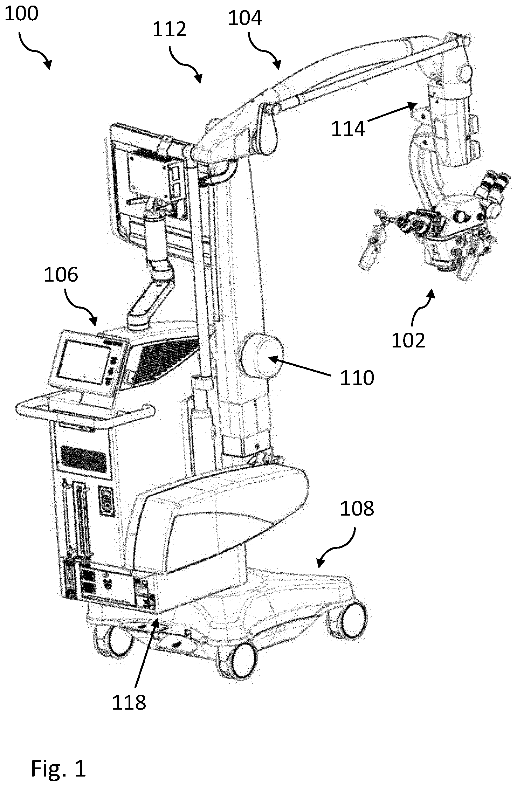

**Fig.** 1 shows a perspective view of a surgical imaging device 100, such as a surgical microscope. In this embodiment, the surgical imaging device 100 comprises an image acquisition apparatus carrier 102, which may be a camera, an optics carrier or the like, an arm 104 for holding the image acquisition apparatus carrier 102, a stand 106, and a base 108. The stand 106 is arranged on top of the base 108. The arm 104 comprises a hollow joint 110 for mounting the arm at the stand 106 and allowing the arm 104 to be movably connected to the stand 106.

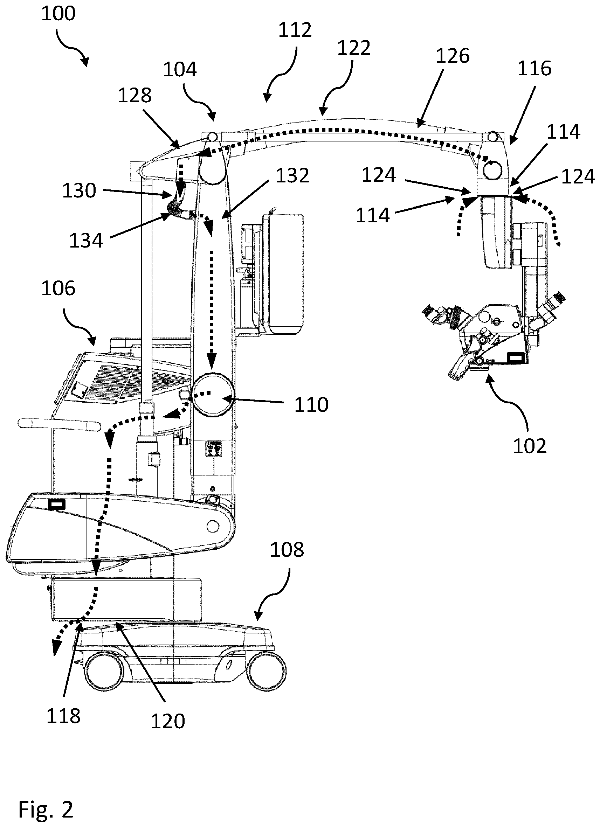

The surgical imaging device 100 further comprises a ventilation system 112, which is configured to generate an air flow inside the surgical imaging device 100. An air intake 114 of the ventilation system 112 is positioned at a first end 116 of the arm 104. An exhaust part 118 of the ventilation system 112 is arranged at a bottom side 120 of the stand 106 facing the base 108 so that the air flow may be directed downwards out of the stand 106 and passing the base 108. It is anticipated that the exhaust air is guided onto a floor of an operating room (not shown) in which the surgical imaging device 100 may be set up. Thereby the exhaust air may not disturb the user while performing medical procedures using the surgical imaging device 100. Within the surgical imaging device 100, the air flow is guided along a flow path 122 of the ventilation system 112, wherein the flow path 122 extends from the air intake 114 though the arm 104 and the stand 106 to the exhaust part 118 (see **Fig. 2**ff for more details).

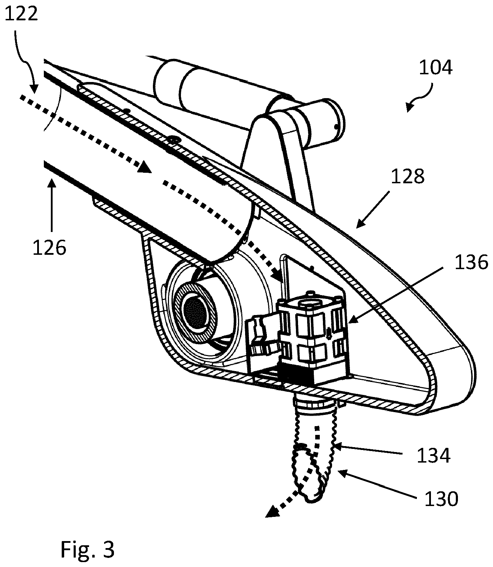

**Fig. 2** shows a side view of the surgical imaging device 100. Dashed lines with arrowheads indicate the air flow along the flow path 122 and its direction within the respective parts of the surgical imaging device 100 (see also **Fig. 3****,** **Fig. 5** and **Fig. 6**)**.** Air is taken up at the air intake 114 at the first end 116 of the arm 104. The air intake 114 comprise two intake ports 124. In further embodiments, the air intake 114 may comprise only one or more than two intake ports 124. This may ensure an even more improved uptake of air for each embodiment. The image acquisition apparatus carrier 102, the arm 104 and the stand 106 may further be configured to be at least partly covered by a drape. The ventilation system 112 may be configured to reduce the air volume inside the drape with the air intake 114 configured to taking up air from the air volume inside the drape. The intake port 124 may thereby connect the air volume inside the surgical imaging device 100 with the volume inside the drape. However, the air intake 114 may not be required to form an airtight barrier as the air flow along the flow path 122 may create a suction force sufficient for taking up air via the intake port 124, thereby avoiding significant backflow of air into the drape. The exhaust part 118 is positioned so that when the arm 104 and/or stand 106 are covered by the drape the exhaust part 118 is not covered by the drape. By that an unintended movement of the drape by the exhaust air, which may disturb the user during a medical procedure, can be avoided.

Following air intake via the intake port 124, the air flow is guided along the flow path 122 inside a first sub-arm 126 of the arm 104, through a connector piece 128 of the arm 104 and via a flexible connection element 130 of the arm into a second sub-arm 132 of the arm 104. The connection element 130 may be a flexible tube 134 attached to the connector piece 128 and the second sub-arm 132. The flexible tube 134 allows for a free movement of the arm 104 without disturbing the air flow. A length of the tube 134 may be adapted to the desired degree of movement of the arm 104. The flow path 122 further extends from the arm 104 through a hollow joint 110 and into the stand 106. The hollow joint 110 may thereby be configured to allow for a sufficient air flow from the arm 104 into the stand 106 so that no further connection element may be required. Furthermore, the air flow is guided through the stand 106 to the exhaust part 118. The exhaust part 118 is arranged at a bottom side 120 of the stand 106 facing the base 108. Through the exhaust part 118 the air being sucked from the air intake 114 through the arm 104 and the stand 106 is guided out of the surgical imaging device 100. In an embodiment, the exhaust part 118 may comprise one or more exhaust ports (not shown).

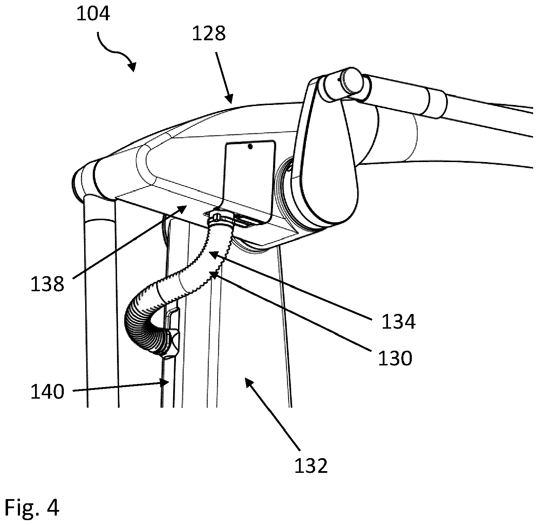

**Fig. 3** and **Fig. 4** show schematic views of the connector piece 128 of the arm 104. The connector piece 128 is movably connecting the first sub-arm 126 and the second sub-arm 132 of the arm 104.

In the embodiment shown in **Fig. 3****,** a fan 136 of the ventilation system 112 is positioned within the connector piece 128. The fan 136 may discharge air into the flexible connection element 130 of the arm 104 thereby guiding the air flow from the connector piece 128 into the second sub-arm 132. The fan 136 is configured to create a suction force sufficient for taking up air by the air intake 114 and moving the air along the flow path 122 within the arm 104. In an embodiment the fan 136 is configured to create the suction force within the whole of the surgical imaging device 100 so that no further fan may be required. In another embodiment, one or more further fan(s) may be placed along the flow path 122 to create the suction force within the surgical imaging device 100. The fan 136 may further be configured to be operated in different operating states, which may be selectable by the user and/or set in advance for operating the surgical imaging device 100.

**Fig. 4** further shows the connection element 130, which is a flexible tube 134 connecting the connector piece 128 and the second sub-arm 132. In this embodiment, the tube 134 is attached at a bottom surface 138 of the connector piece 128 and at a side surface 140 of the second sub-arm 132. The tube 134 is thereby wound around the arm 104 in such way that it allows for a free movement of the arm 104. By that an operation of the surgical imaging device 100, without disturbing the air flow along the flow path 122 is achieved.

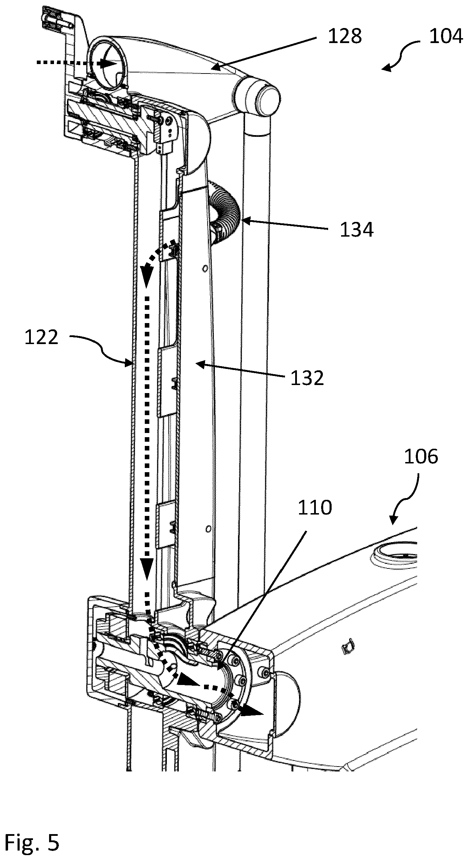

**Fig. 5** shows another schematic section view of the arm 104 at the position of the second sub-arm 132. After being guided from the connection element 130 into the second sub-arm 132, the air flow continues along the flow path 122 through the second sub-arm 132 and into the hollow joint 110, which connects the second sub-arm 132 with the stand 104.

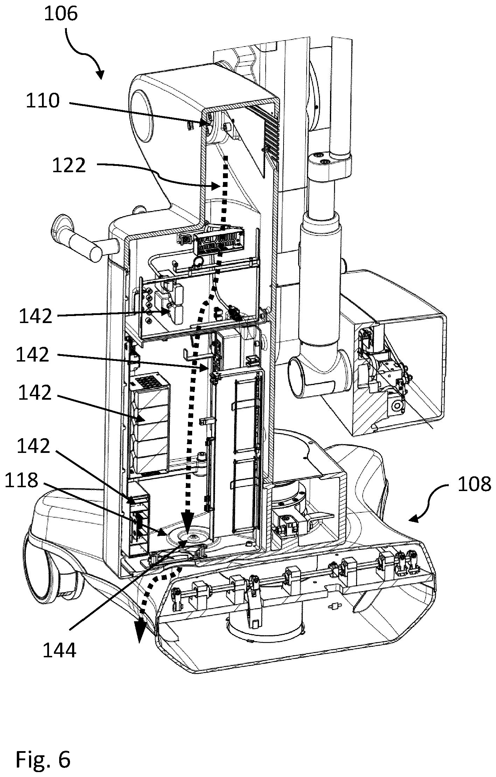

**Fig. 6** shows schematic section view of the stand 106. The stand 106 is positioned on top of the base 108. The flow path 122 is thereby arranged so that the air flow passes heat dissipating electronic components 142 of the surgical imaging device 100, which are positioned inside the stand 106. Electronic components 142 of the surgical imaging device 100 may include processing unit, graphics card, memory cards, etc. used for operating the surgical imaging device 100 and known to a person skilled in the art. The air flow may have a lower temperature compared to a temperature of the electronic components 142 so that the electronic components 142 may be cooled by the passing air flow. For improved guiding of the air flow through the stand 106 and/or via the electronic components 142, the stand 106 may further comprise at least one guiding element (not shown). The guiding element may be a channel, grove, opening, or the like, which may be arranged inside the stand 106.

In the depicted embodiment, the fan 144 of the ventilation system 112 is positioned at the exhaust part 118 and is configured to discharge air through the exhaust part 118 and out of the stand 106. The fan 144 may be a second fan 144 in addition to the fan 136 (not shown). The fan 144 may thereby promote exhaust of air and/or drive the air flow along the flow path 122. In case of the latter, it may be desirable to drive the air flow only or mainly by the second fan 144, especially when a drape is used to partly cover the surgical imaging device 100 and the volume of the drape has been reduced to a desired minimum. Alternatively, both fans 136, 144 may operate simultaneously to drive the air flow along the flow path 122.

In further embodiments, the ventilation system 112 may only comprise one of the fans 136, 144 to drive the airflow along the flow path 122. In one embodiment, only the fan 136, which is then positioned inside the connector piece 128, may be used to drive the air flow along the flow path 122. In another embodiment, only the fan 144, which is positioned at the exhaust part 118, may be used to drive the air flow along the flow path 122. By using one, two or even more fans 136, 144, an optimal configuration of the ventilation system 112 may be achieved for any surgical imaging device 100 or any specific scenario in which a surgical imaging device 100 is being used.

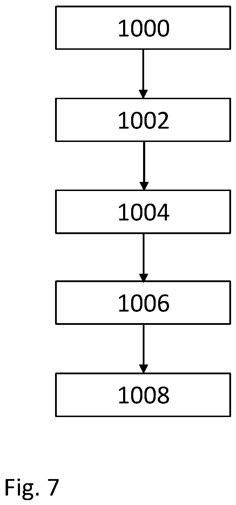

A method for ventilation of a surgical imaging device 100 is shown in **Fig. 7****.** In this embodiment, the method is comprising the following steps: In a first step 1000, surgical imaging device 100 is powered up so that it is ready to be used. It is anticipated that the surgical imaging device may be positioned in an operating room and further be at least partly covered by a drape. In a second step 1002, the fan 136 is being initiated at a high-power state thereby creating an air flow suitable for reducing an air volume inside the drape. In a next step 1004, a desired reduced air volume inside the drape is reached. This may be detected by sensors, which are known to a person skilled din the art. Alternatively, the fan 136 may be set to a speed sufficient to reduce the volume inside the drape, but not too high to adhere the drape too much to the surgical imaging device 100 to block the air flow. It is desired to bring the drape close to surgical imaging device 100 to avoid disturbing the user while operating the surgical imaging device and/or performing a medical procedure. In a subsequent step 1006, when the reduced volume inside the drape had been reached, the fan 136 is switched to a second power state suitable for maintaining the reduced air volume inside the drape. The second power state of the fan and/or a power-on state of a second fan of the ventilation system is thereby suited to maintain the air flow along the flow path. The second state of the fan 136 may be a power-off state in an embodiment, where the second fan is sufficient to perform the air flow maintenance and reduction of the drape volume. Alternatively, the second power state of the fan 136 may be a reduced power state, which may also be used in embodiments without a second fan. The second power state allows for energy saving and noise reduction during a long-term usage of the surgical imaging device 100. In a last step 1008 the surgical imaging device 100 is switched off, including its ventilation system 112. The drape may be removed from the surgical imaging device 100 and cleaned or replaced.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### List of Reference Signs

- 100: Surgical imaging device
- 102: Image acquisition apparatus carrier
- 104: Arm
- 106: Stand
- 108: Base
- 110: Joint
- 112: Ventilation system
- 114: Air intake
- 116: End
- 118: Exhaust part
- 120: Side
- 122: Flow path
- 124: Air intake port
- 126: Sub-arm
- 128: Connector piece
- 130: Connection element
- 132: Sub-arm
- 134: Tube
- 136: Fan
- 138: Surface
- 140: Surface
- 142: Electronic components
- 144: Fan

- 1000: Step
- 1002: Step
- 1004: Step
- 1006: Step
- 1008: Step

## Claims

1. Surgical imaging device (100) comprising an image acquisition apparatus carrier (102), an arm (104), a stand (106), and a ventilation system (112);
wherein the ventilation system (112) comprises an air intake (114), a fan (136, 144), a flow path (122), and an exhaust part (118);
wherein the ventilation system (112) is configured to generate an air flow inside the surgical imaging device (100);
wherein the fan (136, 144) is positioned within the flow path (122) configured to create a directed air flow along the flow path (122) from the air intake (114) to the exhaust part (118); wherein an intake port (124) of the air intake (114) is positioned at a first end (116) of the arm (104);
wherein the exhaust part (118) is positioned in the stand (106);
wherein the flow path (122) extends from the intake port (124) though the arm (104) and the stand (106) to the exhaust part (118).

2. Surgical imaging device according to claim 1, wherein the image acquisition apparatus carrier (102), the arm (104) and the stand (106) are configured to be at least partly covered by a drape; wherein the ventilation system (112) is further configured to reduce an air volume inside the drape with the air intake (114) configured to taking up air from the air volume inside the drape; wherein the exhaust part (118) is positioned so that when the arm (104) and stand (106) are covered by the drape the exhaust part (118) is not covered by the drape.

3. Surgical imaging device according to claim 1 or 2, the surgical imaging device (100) further comprising a base (108); wherein the stand (106) is arranged on top of the base (108); wherein the exhaust part (118) is arranged at a bottom side (120) of the stand (106) facing the base (108) so that the air flow is directed downwards out of the stand (106) and passing the base (108).

4. Surgical imaging device according to one of the preceding claims, wherein the flow path (122) is arranged so that the air flow passes heat dissipating electronic components (142) of the surgical imaging device (100), which are positioned inside the stand (106).

5. Surgical imaging device according to one of the preceding claims, wherein the fan (136) is positioned within a connector piece (128) of the arm (104), with the connector piece (128) movably connecting a first sub-arm (126) and a second sub-arm (132) of the arm (104); wherein the fan (136) discharges air into a flexible connection element (130) of the arm (104) thereby guiding the air flow from the connector piece (128) into the second sub-arm (132).

6. Surgical imaging device according to claim 5, wherein the connection element (130) is a flexible tube (134) attached to the connector piece (128) and the second sub-arm (132).

7. Surgical imaging device according to one of the preceding claims, wherein the fan (144) of the ventilation system (112) is positioned at the exhaust part (118) and is configured to discharge air through the exhaust part (118) and out of the stand (106).

8. Surgical imaging device according to one of the preceding claims, wherein the arm (104) further comprises a hollow joint (110) for mounting the arm (104) at the stand (106); wherein the flow path (122) extends from the arm (104) through the hollow joint (110) and into the stand (106).

9. Surgical imaging device according to one of the preceding claims, wherein the stand (106) comprises at least one guiding element for guiding the air flow inside the stand (106).

10. Surgical imaging system comprising a surgical imaging device according to one of the preceding claims and a detachable drape; wherein the drape is configured to partly cover the surgical imaging device (100).

11. Method for ventilation of a surgical imaging device according to one of the preceding claims, the method comprising:
powering up of the surgical imaging device (100);
initiating the fan (136) at a high-power state thereby creating an air flow suitable for reducing an air volume inside a drape, which is at least partly covering the surgical imaging device (100);
reaching a reduced air volume inside the drape;
switching the fan (136) to a second power state suitable for maintaining the reduced air volume inside the drape;
wherein the second power state of the fan (136) and/or a power-on state of a second fan (144) of the ventilation system (112) is suited to maintain the air flow along the flow path (122).
